(19) Europäisches Patentamt
European Patent Office
Office européen des brevets

(11) Veröffentlichungsnummer: **0 027 159 B1**

(12)

# EUROPÄISCHE PATENTSCHRIFT

(45) Veröffentlichungstag der Patentschrift:
28.03.84

(51) Int. Cl.³: **A 61 F 1/03**

(21) Anmeldenummer: 80104098.1

(22) Anmeldetag: 15.07.80

(54) Hüftgelenkprothese.

(30) Priorität: 11.10.79 CH 9217/79

(43) Veröffentlichungstag der Anmeldung:
22.04.81 Patentblatt 81/16

(45) Bekanntmachung des Hinweises auf die Patenterteilung:
28.03.84 Patentblatt 84/13

(84) Benannte Vertragsstaaten:
AT BE DE FR GB IT NL SE

(56) Entgegenhaltungen:
DE - A - 2 246 940
DE - A - 2 551 013
DE - A - 2 805 305
FR - A - 2 429 010
US - A - 3 863 273

ORTHOPÄDE, Band 8, 1979 M.E. MÜLLER et al.
"Coxarthrose" Seiten 73 bis 74

(73) Patentinhaber: GEBRÜDER SULZER
AKTIENGESELLSCHAFT, Zürcherstrasse 9,
CH-8401 Winterthur (CH)
Patentinhaber: Protek AG, Stadtbachstrasse 64,
CH-3000 Bern (CH)

(72) Erfinder: Müller, Maurice E., Prof. Dr.-med., Inselspital,
CH-3010 Bern (CH)

(74) Vertreter: Dipl.-Ing. H. Marsch Dipl.-Ing. K. Sparing
Dipl.-Phys.Dr. W.H. Röhl Patentanwälte,
Rethelstrasse 123, D-4000 Düsseldorf (DE)

## Hüftgelenkprothese

Die Erfindung betrifft eine Hüftgelenkprothese mit einem geraden, blattartigen Schaft, der sich vom distalen freien Ende zunächst symmetrisch zu einer Längsmittelachse konisch erweitert, wobei die Konusfläche der lateralen Schmalseite auf etwa 3/4 der — entlang der Längsmittelachse gemessenen — Schafthöhe in eine zur Längsmittelachse geneigte Schrägfläche übergeht, während die mediale Schmalseite aus dem Konus heraus in einer stetig gekrümmten Kurve zu einem den Prothesenhals von den Breitseiten des Schaftblattes trennenden Kragen verläuft. Unter «Kragen» wird hierbei im technischen Sinne ein vorspringender Absatz vertanden, der im Gegensatz zum «orthopädischen Kragen» keine abstützende oder tragende Funktion hat, sondern vor allem den Verlauf der Resektionsebene des Schenkelhalses markiert. Die Schafthöhe ist — dem allgemeinen Sprachgebrauch entsprechend — die vertikale Abmessung des Schafts von «Spitze zu Spitze», gemessen entlang seiner Längsmittelachse.

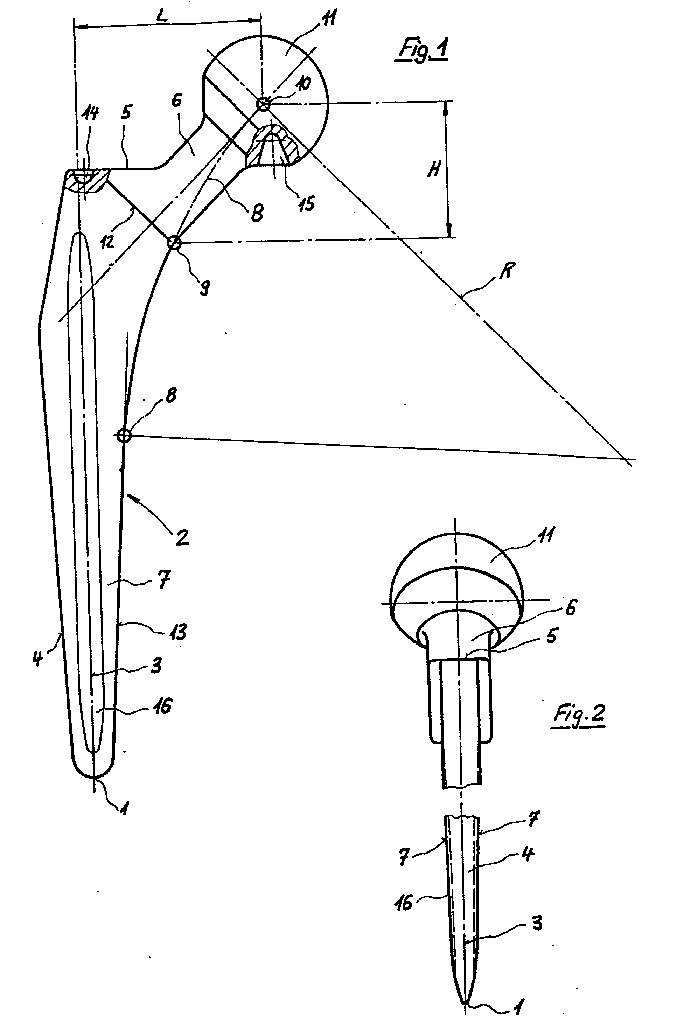

Ein blattartiger Schaft, dessen mediale Schmalseite in einem Kreisbogen zum Kragen zwischen Schaftblatt und Prothesenhals verläuft, ist durch die Prothese nach DE-A1-25 51 013 bekannt. Die DE-A1-22 46 940 zeigt eine Konstruktion der eingangs genannten Art; diese wird mittels eines Knochenzementbettes verankert. Eine gleichartige Konstruktion ist aus der Zeitschrift «Orthopäde» 8 (1979), Seite 73/74, insbesondere Abbildung 1, bekannt. Der sogenannte Geradschaft der Prothese aus der genannten Zeitschrift hat die Aufgabe, sich in dem operativ genau auf seine Dimensionen abgestimmten Hohlraum der Markhöhle zu verklemmen, wodurch ein ihn gegebenenfalls umschliessender Köcher aus Knochenzement weitgehend von tragenden Funktionen entlastet wird; die tragende Abstützung dieser Prothese erfolgt dann in erster Linie durch Verklemmen des Schaftes in der Markhöhle und Abstützen der stetig gekrümmten Kurve der medialen Schmalseite auf dem im Femur medial gelegenen Calcarbogen; diese Kurve ist daher in ihrem Verlauf an einen Mittelwert natürlicher Calcarbogen angepasst. Die Schnittstelle der medialen Schmalseite mit dem Kragen am Uebergang zum Prothesenhals liegt auf der Höhe der Schnittfläche des im wesentlichen senkrecht zur Schenkelhalsachse verlaufenden Operationsschnittes auf dem Calcarbogen auf und legt so die Höhe der Prothese im Körper fest.

Die weitgehende Anspassung des Geradschaftes, der zementfrei oder auch mit Zementköcher implantiert werden kann, an die individuellen Verschiedenheiten der einzelnen Patienten erfolgt mit Hilfe eines Bausatzes gleichartiger Schäfte, die in ihrer Länge bzw. Höhe und ihrer Breite variieren, wobei der Winkel zwischen der Längsmittelachse und der Prothesenhalsachse — um die resultierende Belastung nicht zu vergrössern — und — wegen der Anpassung an die Ansätze der zwischen Becken und Femur verlaufenden Muskulatur — gleichzeitig die Länge des Prothesenhalses konstant gehalten werden, wodurch sich ein konstanter Wert für den in Fig. 1 mit «H» bezeichneten, vertikalen Abstand zwischen dem Mittelpunkt des Gelenkkopfes und der Schnittstelle von Kragen und medialer Schmalseite ergibt. Bei Patienten mit angeborenen oder erworbenen krankhaften Deformationen im Hüftgelenkbereich besteht nun häufig die Notwendigkeit, den in Fig. 1 mit «L» bezeichneten, lateralen Abstand zwischen Gelenkkopfmittelpunkt und Schaftlängsachse der vorstehend beschriebenen Standard- oder Normalprothese zu vergrössern, ohne dass dabei der Schaft verändert wird, d.h. ohne dass insbesondere der Abstand H und der genannte Winkel, sowie der Bogen der medialen Schmalseite variiert werden; denn die Längsachse des Geradschaftes sollte nach der Implantation im wesentlichen mit der Längsachse des Femurs übereinstimmen, um die Verankerung des schaftes im Femur und die Kraftübertragung vom Schaft auf den ihn umgebenden Femurknochen optimal zu gewährleisten.

Mit anderen Worten liegt die Aufgabe der Erfindung darin, für jedes «Individuum» des erwähnten Bausatzes die erwähnte Vergrösserung des lateralen Abstandes unter den geschilderten Voraussetzungen zu erreichen, d.h. zu jedem Exemplar der Standardprothese ein «lateralisiertes» Element zu schaffen. Diese Aufgabe wird dadurch gelöst, dass die stetig gekrümmte Kurve für die mediale Schmalseite ein Kreisbogen ist, der die Uebergangsstelle des Schaftkonus, die Schnittstelle mit dem Kragen und den Mittelpunkt des Gelenkkopfes verbindet.

Mit Hilfe der erfindungsgemässen Massnahme, bei der sich an der Prothese äusserlich lediglich die Länge des Prothesenhalses ändert, gelingt es, den Femurknochen relativ zur Gelenkpfanne des Beckens lateral zu verschieben, ohne den Winkel seiner Längsachse zur Prothesenhalsachse und die erwähnte Höhe H des Gelenkkopfes zur Schnittstelle des Kragens und damit die Höhe des die Prothese abstützenden Calcarbogens relativ zum Becken zu ändern. Für die einzelnen Paare von Normal- und «lateralisierter» Form einer «Grösse» des Bausatzes lässt man bei dem Bogen der medialen Schmalseite Krümmungsradius und Lage des Mittelpunktes ebenfalls konstant; durch die Anwendung unterschiedlicher Radien (R in Fig. 1) für den Kreisbogen der medialen Schmalseite bei einem Bausatz erhält man jedoch die Möglichkeit, die sich nach Einsetzen einer dieser Prothesen ergebende Beinlänge in gewissem Umfang einzustellen.

Einen verbesserten Uebergang der Belastungskräfte vom Kreisbogen der medialen Prothesenschmalseite auf den Femurknochen kann man darüberhinaus erreichen, wenn die Uebergangsstelle der Schaftkonus unterhalb der Mitte der Schafthöhe angeordnet ist. Die geometrische Anpassung der Prothese in engen Grenzen an den einzelnen Patienten erfordert, um gleichzeitig für jeden Patienten einen möglichst weitgehend richtigen Sitz der Prothese zu ereichen, — wie leicht einzusehen ist — ein Einschlagen des Geradschaftes in die operativ erweiterte Markhöhle des Femur mit relativ grosser Genauigkeit. Um daher beim Einschlagen Kippmomente auf den Prothesenschaft weitestgehend zu verhindern, ist es zweckmässig, eine, auf der den Uebergang von der lateralen Schmalseite zum Schenkelhals bildenden Schulter vorgesehene Vertiefung für den Einsatz eines Einschlaginstruments gegenüber der Längsmittelachse des Schaftblattes medial versetzt anzuordnen, wobei die Versetzung, die bis zu 5 mm betragen kann, im allgemeinen 2 - 3 mm gross ist.

Ist es — beispielsweise bei Veränderungen des Knochens im Lauf der Zeit — erforderlich, eine mit einem Geradschaft versehene Prothese gemäss der Erfindung durch eine andere gleichartige zu ersetzen, so wird das Einsetzen der zweiten Prothese erheblich erleichtert, wenn der ursprüngliche Zementköcher oder die von der ersten Prothese geschaffene Ausnehmung in die Markhöhle soweit wie möglich beim Ausschlagen der ersten Prothese unversehrt erhalten bleiben; daher ist es vorteilhaft, wenn die erfindungsgemässe Prothese an der Unterseite des Gelenkkopfes zusätzlich mit einer Ausschlagvertiefung versehen wird, um beim Ausschlagen eine bestimmte Ausschlagrichtung soweit wie möglich festzulegen.

Im folgenden wird die Erfindung anhand eines Ausführungsbeispiels im Zusammenhang mit der Zeichnung näher erläutert.

Fig. 1 zeigt die neue Prothese in einer Aufsicht auf das Schaftblatt, während

Fig. 2 eine Ansicht von Fig. 1 von links darstellt.

Vom distalen Ende 1 aus erweitert sich der blattartige Schaft 2, zunächst symmetrisch zu seiner Längsmittelachse 3, konisch. Etwa auf 3/4 der Schafthöhe, die von «Spitze zu Spitze» entlang der Längsmittelachse 3 gemessen wird, besitzt die laterale Schmalseite 4 des Schaftes eine Unstetigkeit, an der der sich vom distalen Ende 1 erweiternde Konus in eine zur Längsmittelchse 3 hin verlaufende Schrägfläche übergeht. Diese endet in einer mindestens nahezu horizontalen Schulter 5, die den Uebergang zum Schenkelhals 6 bildet.

Etwa auf halber Schafthöhe geht der sich erweiternde Konus der medialen Schmalseite 13 des Schaftblattes 7 in einen Kreisbogen B über. Dieser verbindet die Uebergangsstelle 8 mit einem Punkt 9, in dem der Kreisbogen die Verlängerung des den Schaft 2 vom Prothesenhals 6 trennenden, kragenartigen Absatzes 12 schneidet. Der Punkt 9 ist die erwähnte Schnittstelle der medialen Schmalseite 13 mit dem Kragen 12, die — wie eingangs erwähnt — zu den ausgezeichneten Fixpunkten bei der Verankerung des Schaftes im Femurknochen gehört.

Vom Punkt 9 aus setzt sich der Kreisbogen B erfindungsgemäss fort bis zum Mittelpunkt 10 des Gelenkkopfes 11. Sein Radius R ist konstruktiv an den Verlauf natürlicher Calcarbogen angepasst. Der laterale Abstand des Mittelpunktes 10 von der Längsmittelachse 3 des Schaftblattes 7 ist mit L und die Höhe, die den vertikalen Sollabstand der Punkte 9 und 10 bildet, mit H bezeichnet.

Auf der Schulter 5 ist zur Längsmittelachse 3 medial verschoben eine Vertiefung 14 für den Einsatz eines Einschlaginstrumentes vorgesehen. Die mediale Verschiebung der Einschlagvertiefung 14 bewirkt, dass beim Einschlagen auf den Schaft evtl.

einwirkende Kippmomente möglichst verringert werden und allenfalls ein Kippmoment in Richtung auf den Calcarbogen an der medialen Seite des Femurs ausgeübt wird, wodurch ein fester Sitz des stetig gekrümmten Teils der Schmalseite 13 auf dem Knochen erreicht wird.

An der Unterseite hat der Gelenkkopf 11 eine Ausschlagvertiefung 15, mit deren Hilfe, wie ebenfalls bereits erwähnt, beim Ausschlagen eine bestimmte Richtung eingehalten werden kann.

Am distalen Ende 1 sind die Blattseiten 7, in denen Längsnuten 16 vorgesehen sind, durch einen kreisförmigen Uebergang von der Schmalseite 4 zur Schmalseite 13 abgeschlossen, während sie senkrecht dazu mit relativ grossen Radien in einer Spitze auslaufen (Fig. 2). Die Krümmung dieses Auslaufens ist dabei so gewählt, dass möglichst ein stetiger Uebergang des belastenden Kraftflusses vom Schaft 2 auf den umgebenden Zementköcher und/oder das — unter Umständen beim Einschlagen der Prothese verdichtete — Knochengewebe erfolgt.

## Patentansprüche

1. Hüftgelenkprothese mit einem geraden, blattartigen Schaft (2), der sich vom distalen freien Ende (4) zunächst symmetrisch zu einer Längsmittelachse (3) konisch erweitert, wobei die Konusfläche der lateralen Schmalseite (4) auf etwa 3/4 der — entlang der Längsmittelachse (3) gemessenen — Schafthöhe in eine zur Längsmittelachse (3) geneigte Schrägfläche übergeht, während die mediale Schmalseite (13) aus dem Konus heraus in einer stetig gekrümmten Kurve zu einem, den Prothesenhals (6) von den Breitseiten des Schaftblattes (7) trennenden Kragen (12) verläuft, dadurch gekennzeichnet, dass die stetig gekrümmte Kurve für die mediale Schmalseite (13) ein Kreisbogen (B) ist, der die Uebergangsstelle (8) des Schaftkonus, die Schnittstelle (9) mit dem Kragen (12) und den Mittelpunkt (10) des Gelenkkopfes (11) verbindet.

2. Hüftgelenkprothese nach Anspruch 1 dadurch gekennzeichnet, dass die Uebergangsstelle (8) des Schaftkonus unterhalb der Mitte der Schafthöhe angeordnet ist.

3. Hüftgelenkprothese nach Anspruch 1, dadurch gekennzeichnet, dass eine, auf der den Uebergang von der lateralen Schmalseite (4) zum Prothesenhals (6) bildenden Schulter (5) vorgesehene Vertiefung (14) für den Einsatz eines Einschlaginstrumentes gegenüber der Längsmittelachse (3) des Schaftblattes (7) medial versetzt angeordnet ist.

4. Hüftgelenkprothese nach Anspruch 3, dadurch gekennzeichnet, dass an der Unterseite des Gelenkkopfes (11) zusätzlich eine Ausschlagvertiefung (15) vorgesehen ist.

## Claims

1. A hip joint prosthesis having a stright blade-like stem (2) which widens conically from the distal free end (4) initially symmetrically of a longitudinal central axis (3), the cone surface of the lateral narrow side (4) merging, about threequarters of stem height as measured along said axis (3), into an inclined surface which is at an inclination to said axis (3), whereas the medial narrow side (13) extends out of the cone in a continuous curve to a collar (12) which separates the prosthesis neck (6) from the wide sides of the blade (7), characterised in that the continuous curve for the medial narrow side (13) is a circular arc (B) interconnecting the transition zone (8) of the stem cone, the point of intersection (9) with the collar (12) and the centre (10) of the joint head (11).

2. A prosthesis according to claim 1, characterised in that transition zone (8) of the stem cone is disposed below the centre of the stem height.

3. A prosthesis according to claim 1, characterised in that a recess (14) in the shoulder (5) forming the transition from the lateral narrow side (4) to the neck (6) and serving for the introduction of a striking instrument is offset medially from the stem blade axis (3).

4. A prosthesis according to claim 3, characterised in that the underside of the joint head (11) is also formed with a knocking-out recess (15).

**Revendication**

1. Prothèse d'articulation de la hanche comprenant une tige (2) rectiligne de type lame qui s'élargit coniquement depuis l'extrémité libre distale (4) tout d'abord symmétriquement vers un axe longitudinal (3), la face conique du côté latéral étroit (4) se transformant alors sur à peu près les 3/4 de la hauteur de la tige, mesurée le long de l'axe longitudinal (3), en une face oblique inclinée sur l'axe longitudinal (3), tandis que le côté étroit médian (13) s'étend depuis le cône en une courbe à courbure constante vers un collet (12) séparant le col de la prothèse (6) des côtes larges de la lame (7) de la tige, caractérisée en ce que la courbe à courbure constante pour le côté étroit (13) médiant est un arc de cercle (B) qui relie l'endroit de transition (8) du cône de la tige, l'endroit d'intersection (9) avec le collet (12) et le point central (10) de la tête d'articulation (11).

2. Prothèse d'articulation de la hanche selon la revendication 1, caractérisée en ce que l'emplacement de transition (8) du cône de la tige se trouve en dessous du milieu de la hauteur de la tige.

3. Prothèse d'articulation de la hanche selon la revendication 1, caractérisée en ce qu'une cavité (14) destinée à l'introduction d'un instrument d'enfoncement et prévue sur l'épaulement (5) formant la transition du côté latéral étroit (4) au col de la prothèse (6) est disposée de façon à être décalée par rapport au centre vis-à-vis de l'axe longitudinal (3) de la lame di tige (7).

4. Prothèse d'articulation de la hanche selon la revendication 3, caractérisée en ce qu'on prévoit sur le côté inférieur de la tête d'articulation (11) une cavité additionnelle d'extraction (15).

Fig. 1

Fig. 2